# EUROPEAN PATENT APPLICATION

(11) **EP 4 218 670 A1**
(43) Date of publication of application: **02.08.2023**
(21) Application number: 20954331.3
(22) Date of filing: 25.09.2020
(51) Int. Cl.: A61F 2/14

(54) **DOUBLE-SEGMENT INTRASTROMAL DEVICE FOR CORRECTING SEVERE AMETROPIA**

(71) Applicant: Ferrara De Almeida Cunha, Paulo, Condomínio Quintas da Casa Branca Brumadinho MG (BR)
(72) Inventor: Ferrara De Almeida Cunha, Paulo, Condomínio Quintas da Casa Branca Brumadinho MG (BR)
(74) Representative: Sahuquillo Huerta, Jesús
(86) International application number: PCT/BR2020/050381
(87) International publication number: WO 2022/061425

(57) **Abstract**

The present technology, which falls within the technical field of ophthalmology, more precisely within the field of methods or devices for treating ametropias, is configured as a two-segment intracorneal ring. The invention is based on the use of segments of different shapes and diameters, inserted in the stroma, which induce a significant flattening of the cornea, being able to correct high degrees of myopia and advanced keratoconus, above 60 dioptres. The segment (AFR) presents a trapezoidal or triangular cross section and is inserted in the inner part of the cornea, while (Hm) presents an elliptical cross section, with eccentricity close to zero, inserted in the external region of the cornea. The use of this technology entails a significant reduction in the indications of patients requiring corneal transplantation.

## Description

### Field of Application

The present invention patent refers to a two-segment intracorneal ring. Inserted in the technical field of ophthalmology, more precisely in the field of methods or devices for treating ametropias, the invention is based on the use of two segments of different diameters, inserted in the stroma, which induce a significant flattening of the cornea, being able to correct high degrees of myopia and advanced keratoconus, above 60 dioptres. The use of the segments results in a significant reduction of indications for cornea transplant.

### Description of the state of the art

Non-inflammatory corneal disorders, such as keratoconus, are characterized by progressive thinning of its thickness and are among the most common refractive abnormalities that surgeons encounter. As the thinning progresses, the cornea becomes more irregular. The therapeutic options for patients with this disorder have been limited to spectacles and contact lenses, while in advanced stages of the disease the accepted approach involves surgical interventions and even corneal transplants.

Keratoconus is a corneal ectasia, generally bilateral and asymmetric, with an incidence of one case per two thousand individuals in the population. In severe cases, surgical interventions tend to be avoided as they may lead to a series of complications such as: primary or secondary failure, rejection, infectious keratitis, persistent de-epithelialization, and mainly high astigmatism. Intracorneal rings, for keratoconus correction, may be an alternative for these patients that do not tolerate the use of contact lenses and do not wish to face the risks of a cornea transplant.

The use of intracorneal rings was first proposed by a Russian doctor, Blavatskaya who in 1963 used rings made of corneal tissue for the correction of high myopias.

Changes in corneal structure induced by additive technologies can be predicted by Barraquer's thickness law; that is, when material is added to the periphery of the cornea or an equal amount of material is removed from the central area, a flattening effect is obtained. In contrast, when material is added to the centre or removed from the periphery of the cornea, surface curvature is increased. The corrective result varies in direct proportion to the thickness of the implant and in inverse proportion to its diameter. The thicker and smaller the device, the greater the corrective result.

These rings are usually made of rigid poly (methyl methacrylate) PMMA that are implanted inside the corneal stroma to induce a change in the geometry and refractive power of the tissue. There are 3 main models of intracorneal rings on the market: Ferrara ring; Bisantis segments; and intrastromal rings, being commonly used the commercial brand named Intacs.

Both Intacs and Ferrara rings use a fixed curvature base with respect to the corneal curvature, which generates different effects on astigmatism induction. Thus, the Intacs stand out for not causing astigmatic induction, unlike the Ferrara rings, which may annul high astigmatism present in corneal deformities and ectasias. For this reason, the Ferrara rings are more appropriate for the treatment of these pathologies. However, these induced astigmatisms are sometimes so high that they not only counteract the pre-existing astigmatism but induce an astigmatism inverse to the pre-existing one.

Among the advantages of the Ferrara rings implantation are the fast post-surgery recovery, the absence of the possibility of rejection by the organism and the minimum risk of complications.

Another great advantage of the Ferrara ring surgery is that it is a totally reversible procedure, which enables the implants to be removed, exchanged, or adjusted, when necessary, as in cases where there is inadequate vision correction.

The use of intracorneal implants for ametropia correction is an old practice, described by some authors in the early 1960s, so it is possible to find a number of products, methods and patents involving these materials and their applications, as can be seen in the applications below.

PI 0404515-7 describes an implant in the form of an intracorneal ring for correction of ametropias which, due to a new shape, allows improved quantification of the desired correction, increasing the predictability of the technique and improving the quality of the optical system of the eye. According to the invention, the ring, in the shape of an arc segment between 90 and 360 degrees, made of inert material and biocompatible with the cornea, has a cross-section in the shape of a scalene triangle whose base is the side or major Cetus and whose minor Cetus corresponds to the external face of the ring.

BR 1020160160804 provides a device that will allow the correction of the corneal curvature of patients who present irregular corneas, such as astigmatism induced after corneal transplantation and ectatic diseases such as keratoconus, pellucid or keratoglobus, therefore, the device will comprise of a complete circle, characterized by a central ring, allowing high resistance and strength; attached to two handles and, aided by three modified spatulas for inserting the device at the moment of implantation, and thus obtaining a desired effect as a skeleton, correcting the shape of the cornea.

BR 11 2018 005256 6 presents a method for laser treatment and cutting of a human eye comprising: determining position information of a pupil centre of the eye relative to a minimum corneal thickness point in an undeformed state of the eye; locating the minimum corneal thickness point in a flattened state of the eye, wherein the eye is deformed by contact with a patient adapter of a laser device; and aligning a pulse firing pattern for laser radiation pulses from the laser device based on a position of the localized minimum corneal thickness point and the determined position information.

BR 1020150202318 discloses a correction principle capable of promoting a new corneal structuring, decreasing and stabilizing its central curvature, reducing or eliminating image and light distortions, therefore increasing the quality and visual capacity of the diseased eye, besides preventing the progression of corneal diseases; the invention consists of pre-formed inserts, obtained from biocompatible materials, notably polymeric ones, all equally with a prismatic geometry and elongated in the form of bars, as also they are implanted in the corneal stroma (T) in depth of 75% of its thickness and according to a radial distribution preserving an intact optical zone.

JP2007151768A discloses a ring-shaped structure (spacer) placed in a corneal incision created in a particular pattern and based on a new theory of eyeball fitting. The cross-section of the intracorneal ring is formed into an inverted triangle that narrows towards the outer side.

### State of the art analysis

Despite the large number of technologies involving intracorneal lenses, it is possible to observe a number of distinctions between the prior art and the present invention. The proposals found generally involve modifications to the structure and shape of the lens, in addition to methods for inserting them.

Despite these variations, the ametropia correction field is limited, below 60 dioptres, and higher corrections are not possible with only one segment of the ring. Moreover, due to the nanometric dimensions of the lenses and the small range for a correct and effective positioning in the cornea, the insertion of two segments is not a trivial task, requiring a complete evaluation of the patient's eye topography and high-precision equipment to perform the implantation.

The femtosecond laser, used in this technique, makes the process predictable and safe, because it presents great precision in making tunnels and cuts in the cornea, which was not possible with the manual technique. Without this type of equipment, the insertion of two segments becomes unfeasible.

### Brief description of the objective

In order to develop a two-segment prosthesis that results in a corneal flattening superior to the existing products in the state of the art, for corrections of high degrees of myopia and keratoconus above 60 dioptres, and thus decrease the number of patients requiring corneal transplantation, the present invention was proposed. Its functionalities can be better understood by the following detailed description in line with the figures in the annex, where:
Figure 1 represents the outer ring segment with elliptical cross section, called (Hm).
Figure 2 refers to the inner ring segment with trapezoidal or triangular cross section, called (AFR).

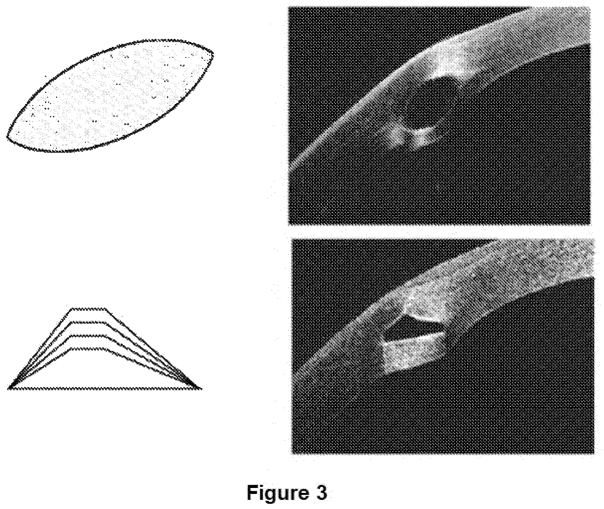

Figure 3 shows the possible variations in the dimensions of the cross-sections of the rings, concomitant with their images after application to the cornea.

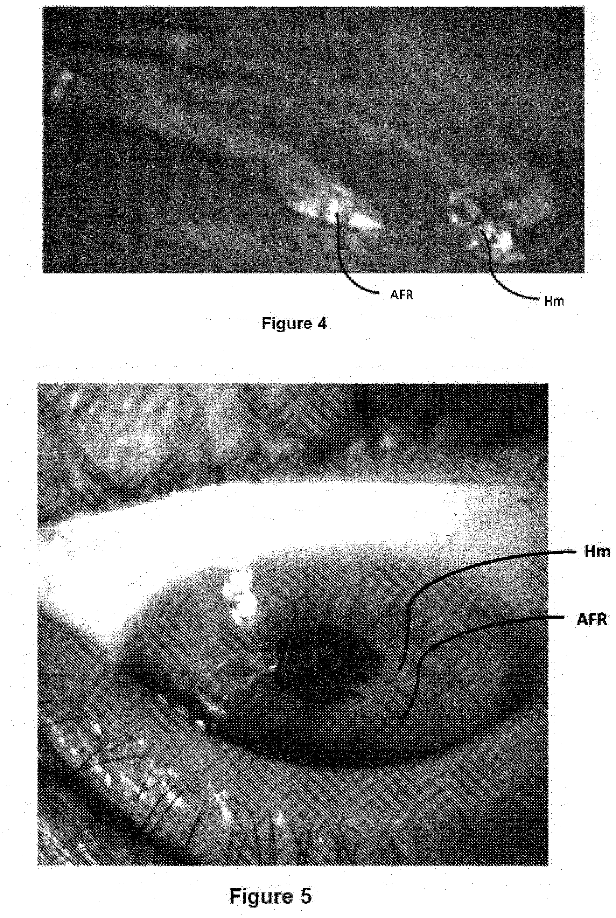

Figure 4 reveals a photo of the two rings to be implanted, in which the external one has an elliptical cross section and the internal one a trapezoidal cross section.
Figure 5 identifies a photo of the two rings (AFR) and (Hm) already implanted in the patient.

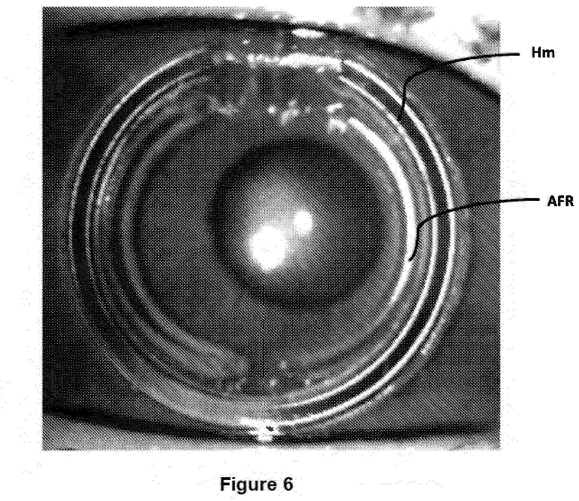

Figure 6 identifies another photo of the two rings (AFR) and (Hm) already implanted in the patient.

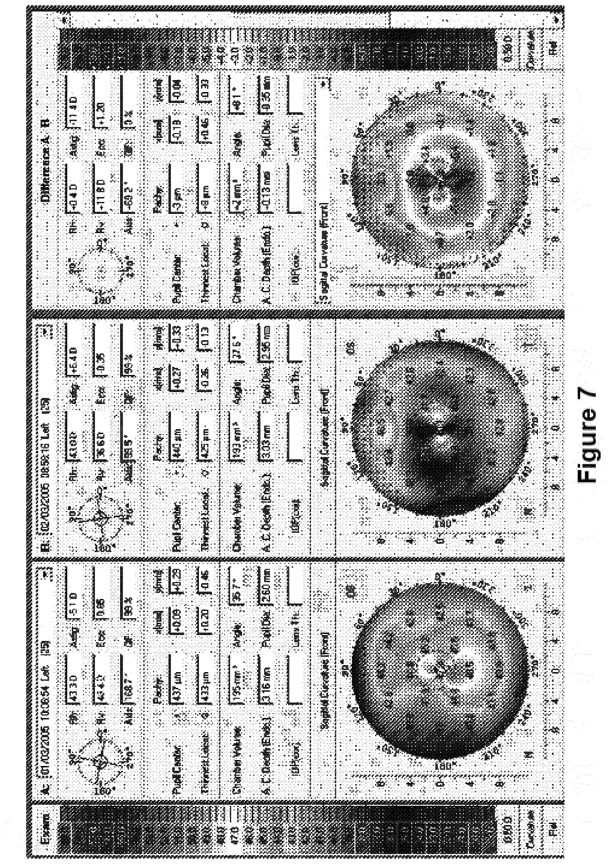

Figure 7 shows the results obtained from the dioptric correction of a patient, comparing the before and after surgery.

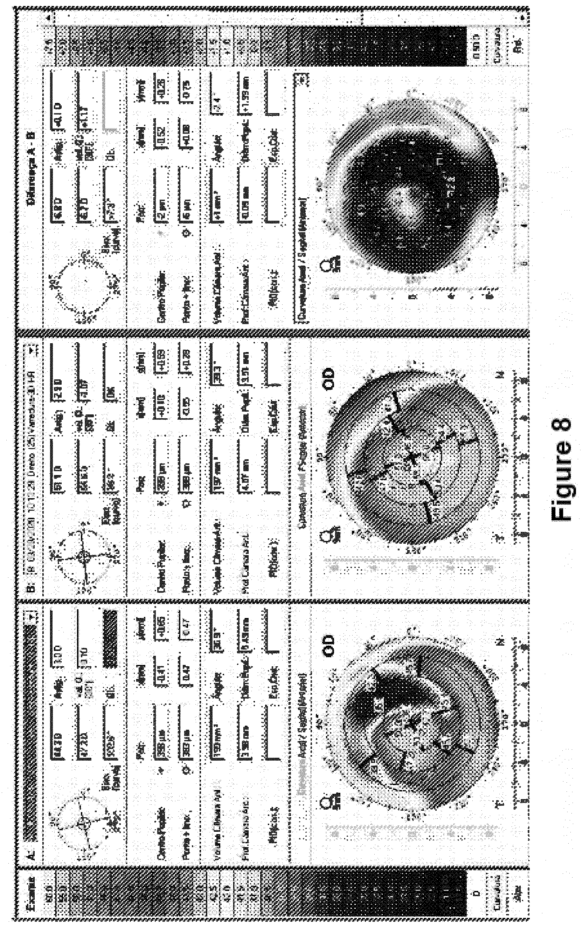

Figure 8 shows the results obtained from the correction of dioptres of another patient, comparing the before and after surgery.

### Detailed description of the invention

Intracorneal or intrastromal rings, are actually rigid semicircular segments of polymethylmethacrylate (PMMA), or any other biocompatible material with suitable optical properties, which are placed in the corneal stroma to modify the curvature of the inner face of the cornea.

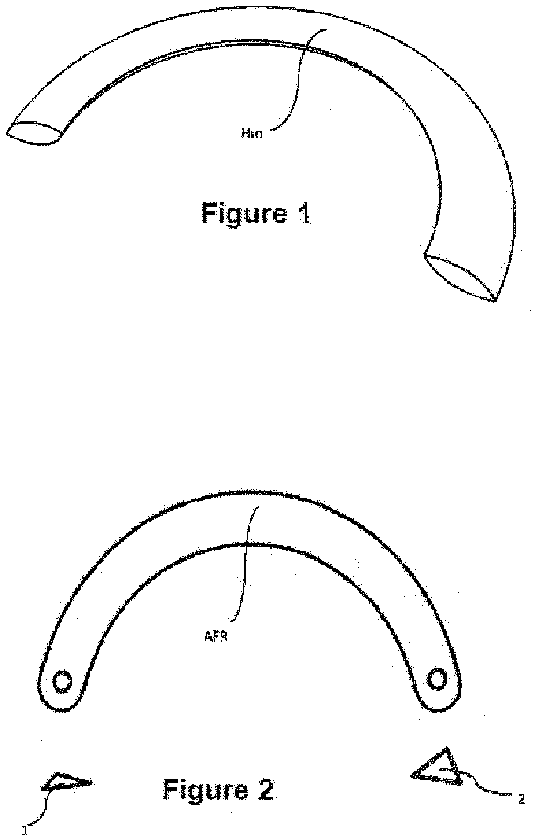

The ring (AFR) presents a trapezoidal cross section, and may also have a triangular cross section, scalene type, i.e., with unequal sides. Figure 2 shows two of these examples (1,2). Once implanted, the ring follows the cornea curvature, independently of these variations.

The ring (Hm) already has an elliptical cross section, with varied eccentricity, so that it results in a better corneal flattening of the patient.

According to the patient's needs, the curvature of the ring base is also chosen (i.e. the cone angle of the ring base that is intended to tangent the cornea at the implant line) and, as a result, the outer diameter of the ring is determined.

The trapezoidal or triangular ring (AFR) should be implanted in the 4mm plane and the elliptical segment (Hm) in the 6mm plane. The depths are calculated in 75% of the thinnest point of the pachymetry of the plane in which the ring will be implanted. Ideally, the differences between them should be at least 50 microns.

The rings (Hm) and (AFR) should be anatomically different. Where the outer segment is elliptical and the inner segment triangular, or trapezoidal, so that they can fit together.

The femtosecond laser or any other device capable of producing a circular lamellar cut in the cornea should be used in this operation. Through the ultra-fast pulse of the laser it is possible to create intracorneal tunnels. These tunnels are used to place the intrastromal rings. The incisions must be offset.

The use of two segments of different diameters results in a great flattening of the cornea, which means correction of high degrees of myopia and very advanced keratoconus, above 60 dioptres, leading to a significant reduction of indications for cornea transplant.

As seen above, there are possibilities of modifications and adaptations according to each case, and the descriptive memory of the present document should not be considered as limiting, but as illustrative, as there may be constructive variations, for those skilled in the technique, that are equivalent without, however, departing from the scope of protection of the invention.

## Claims

1. INTRASTROMAL DOUBLE SEGMENT DEVICE FOR CORRECTIONS OF GRAVIOUS AMETROPIA, known as intracorneal or intrastromal rings, in the form of rigid semicircular segments made of polymethylmethacrylate (PMMA), or any other biocompatible material with adequate optical properties, in which the use of two segments of different diameters results in a great flattening of the cornea, correcting high degrees of myopia and advanced keratoconus, above 60 dioptres, resulting in an expressive reduction of indications for cornea transplant, **characterized by** having an external segment (Hm) and an internal segment (AFR), implanted in the cornea, spaced at least fifty micrometres apart.

2. INTRASTROMAL DOUBLE SEGMENT DEVICE FOR CORRECTIONS OF GRAVIOUS AMETROPIA, according to claim 1, **characterized by** the ring (Hm) having an elliptical cross section, with varied eccentricities.

3. INTRASTROMAL DOUBLE SEGMENT DEVICE FOR GRAVIOUS AMETROPIA CORRECTIONS, according to claim 1, **characterized by** the ring (AFR) having a trapezoidal, or triangular cross section.
